# FASCICULE DE BREVET EUROPEEN

(11) **EP 3 126 057 B1**
(45) Date de publication et mention de la délivrance du brevet: **23.09.2020**
(21) Numéro de dépôt: 15703799.5
(22) Date de dépôt: 12.02.2015
(51) Int. Cl.: B05B 7/16, A61L 9/14, B05B 7/00, B05B 7/24

(54) **DISPOSITIF ET PROCÉDÉ POUR LA DIFFUSION D'UN BROUILLARD SEC**
VORRICHTUNG UND VERFAHREN ZUR DIFFUSION EINES TROCKENNEBELS
DEVICE AND METHOD FOR DIFFUSING A DRY MIST

(30) Priorité: 12.02.2014 FR 1451102
(43) Date de publication de la demande: 08.02.2017
(73) Titulaire: Presensia, 75002 Paris (FR)
(72) Inventeur: SUISSA, David, 94300 Vincennes (FR); MARTIN, Laurent, 93100 Montreuil (FR)
(74) Mandataire: Ipside
(86) Numéro de dépôt international: PCT/EP2015/053034
(87) Numéro de publication internationale: WO 2015/121390

(56) Documents cités:
- EP-A2- 0 261 649
- FR-A1- 2 870 128
- GB-A- 525 736
- US-A- 4 049 200

## Description

L'invention concerne un dispositif et un procédé pour la diffusion d'un brouillard sec. L'invention est plus particulièrement, mais non exclusivement, adaptée à la diffusion, dans un grand volume, d'un parfum en aérosol, stocké sous forme liquide.

Selon l'art antérieur une telle diffusion est obtenue notamment par l'intermédiaire d'un circuit de ventilation à grand débit, telle une climatisation. Le parfum à diffuser se trouve sous forme liquide, dans un récipient comprenant une tête de diffusion débouchant dans ledit circuit de ventilation. Un compresseur permet d'injecter de l'air comprimé dans ladite tête de diffusion, qui par un dispositif de venturi aspire le parfum dans le récipient et le nébulise sous forme de gouttelettes. À la sortie de la tête de diffusion, selon ce procédé de l'art antérieur, les gouttelettes ont un diamètre compris entre 0,5 µm et 100 µm. Les fines gouttelettes sont emportées par le flux de la ventilation et les plus grosses retombent dans le circuit de ventilation, où elles finissent par s'évaporer sous l'effet de ce même flux d'air. Le débit d'air dans une telle installation de ventilation ou de climatisation est supérieur au débit de produit nébulisé dans un rapport de l'ordre de 10⁷.

Toutefois, lorsque l'espace dans lequel le produit doit être diffusé ne comporte pas un circuit de ventilation apte à générer un flux d'air suffisamment puissant, ou encore, lorsque la nature du produit diffusé interdit son utilisation dans les gaines de ventilation ou de climatisation, la nébulisation assurée par la tête de diffusion ne permet pas à elle seule, de diffuser le produit de manière efficace. En effet, une bonne diffusion du produit, notamment un parfum, est obtenue lorsque les gouttelettes nébulisées sont majoritairement vaporisées, c'est-à-dire que lesdites gouttelettes se transforment en vapeur au contact d'un volume d'air non saturé important, et que les gouttelettes non vaporisées sont suffisamment fines pour rester en suspension dans l'air. Pour que ces conditions soient réunies, le diamètre des gouttelettes diffusées doit être inférieur à 10 µm, ce qui permet de diffuser le produit sous la forme d'un brouillard dit sec. Ces conditions ne sont pas obtenues directement en sortie de la tête de diffusion.

Pour pallier cet inconvénient, les dispositifs de l'art antérieur utilisent une chambre, dite de fractionnement, dont l'objet est de trier les gouttelettes afin de ne permettre la diffusion que des gouttelettes les plus fines. Le document EP 0608176 décrit un exemple d'application d'une telle chambre de fractionnement. Le jet de parfum nébulisé est projeté dans une chambre lui imposant un parcours comprenant des obstacles entre l'entrée du jet dans ladite chambre et sa sortie de la chambre de fractionnement. L'impact du jet progressant dans la chambre sur la surface des obstacles, permet de concentrer les très grosses gouttelettes qui ruissellent par gravité le long des parois de la chambre. En effet, les fines gouttelettes tendent à rebondir sur les obstacles sans les mouiller, alors que les gouttelettes d'un calibre plus important éclatent au contact de l'obstacle et mouillent la paroi correspondante. Le jet, ralenti par ce parcours accidenté et réduit en volume par le processus de tri des gouttelettes, quitte la chambre pour l'espace dans lequel il est diffusé, le plus souvent par un petit tuyau permettant de guider le flux vers une sortie à un endroit précis.

Lorsqu'il s'agit de diffuser un produit, notamment un parfum, dans un local, notamment un local commercial, le système de diffusion est avantageusement installé dans un faux plafond. Bien souvent, les contraintes d'encombrement qui en résultent ne permettent pas d'installer une chambre de fractionnement suffisante pour obtenir un tri effectif des gouttelettes, mais ralentissent néanmoins le flux de manière significative. En effet, l'efficacité du dispositif impose que le jet soit émis verticalement. La hauteur du flacon contenant le produit à diffuser étant de l'ordre de 30 cm et l'espace au-dessus du faux plafond étant généralement compris entre 40 cm et 1 mètre, il reste peu de place pour l'installation d'une chambre de fractionnement. De plus, la direction de diffusion du produit dans l'espace, vers le bas lorsque le dispositif est monté dans un faux plafond, rend plus complexe la maîtrise de l'écoulement des gouttelettes interceptées par les obstacles de la chambre de fractionnement. Il en résulte plusieurs inconvénients, parmi lesquels :
- des gouttes du produit diffusé se condensent à la sortie du tuyau, ou à la sortie de la tête de diffusion. Une goutte finit par tomber sur une surface à proximité de la sortie, dans l'espace faisant l'objet de la diffusion du produit, où ladite goutte vient souiller les sols, les murs ou les objets présents dans cet espace ;
- le produit diffusé se condense à l'intérieur du tuyau de sortie, ce qui nuit à la qualité de diffusion, le brouillard passant alors à travers un volume de parfum liquide ;
- la faible vitesse du fluide en sortie homogénéise mal le produit diffusé dans l'espace, ce qui provoque, par exemple, une puissance olfactive trop importante, voire incommodante, à proximité du diffuseur, et non perceptible dans des espaces plus éloignées ;
- le brouillard à la sortie du diffuseur est épais et très visible, ce qui le rend assez anxiogène pour les personnes en présence, et contamine les éléments de l'environnement proche.

Le document EP 0 261 649 décrit un dispositif pour la diffusion d'un brouillard sec, utilisant un ensemble d'ailettes devant le nébulisateur, lesquelles ailettes ont un effet technique et les mêmes inconvénients qu'une chambre de fractionnement. D'autres dispositifs exemplaires sont décrit dans GB 525 736 A et US 4 094 200 A.

L'invention vise à résoudre les inconvénients de l'art antérieur et concerne à cette fin un boîtier, dit anti-condensation selon la revendication 1.

L'invention est avantageusement mise en œuvre selon les modes de réalisation des revendications 2 à 5, lesquels sont à considérer individuellement ou selon toute combinaison techniquement opérante.

L'invention concerne également un dispositif pour la diffusion d'un produit sous la forme d'un brouillard sec selon la revendication 6 et les modes de réalisation des revendications 7 à 9. Ce dispositif de diffusion est autonome et permet de diffuser le produit, notamment un parfum, sans les inconvénients de l'art antérieur, en l'absence de circuit de ventilation ou de climatisation.

L'invention est exposée ci-après selon ses modes de réalisation préférés, nullement limitatifs, et en référence aux figures 1 à 7 dans lesquelles :
- la figure 1 montre selon une vue en perspective et en éclaté de face et de droite, un exemple de réalisation d'un ensemble de diffusion selon l'invention ;
- la figure 2 est une vue en perspective de l'ensemble de la figure 1 selon un point de vue de face et de gauche ;
- la figure 3 représente, selon une vue de droite, l'installation d'un ensemble de diffusion ne faisant pas partie de l'invention dans un habillage représenté en coupe sur cette vue ;
- la figure 4, illustre selon une vue en perspective un autre mode de réalisation de l'ensemble de diffusion ne faisant pas partie de l'invention dans lequel le conduit comprend une partie orientable ;
- la figure 5 représente schématiquement un exemple de réalisation ne faisant pas partie de l'invention, d'un dispositif de diffusion, comprenant une pluralité d'ensembles de diffusion connectée à un bloc pompe unique ;
- la figure 6 est un logigramme d'un exemple de réalisation du procédé de mise en oeuvre ne faisant pas partie de l'invention;
- et la figure 7 illustre selon une vue de profil, un exemple de réalisation ne faisant pas partie de l'invention d'un dispositif comprenant un conduit à double cintre.

Dans tout le texte, le terme « vapeur » désigne la phase gazeuse d'un composé. Les termes « brouillard sec » désignent un mélange diphasique mêlant phases gazeuses et phases liquides dans lequel les phases liquides se trouvent sous la forme de microgouttelettes dont le diamètre est suffisamment réduit pour leur conférer une capacité à rebondir sur les surfaces solides sans mouiller lesdites surfaces.

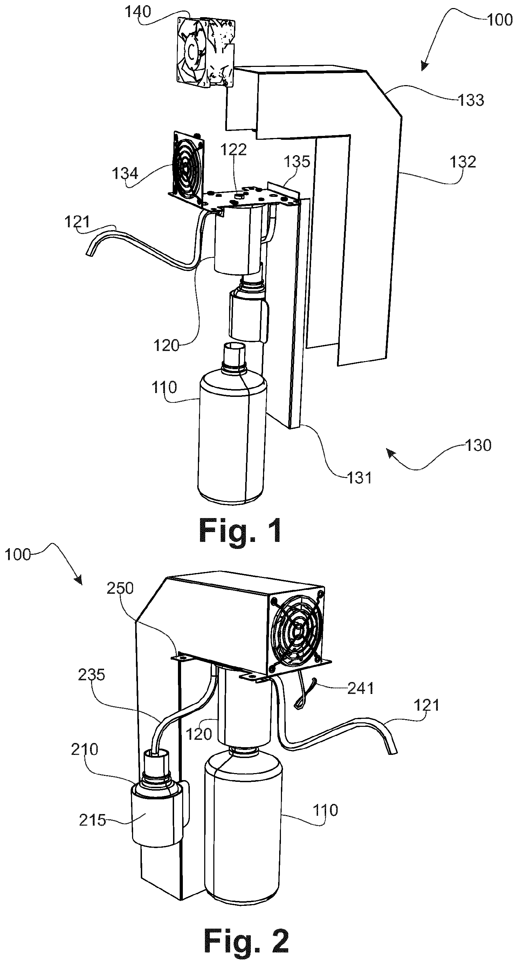

Figure 1, selon un exemple de réalisation, l'ensemble (100) de diffusion objet de l'invention comprend un flacon (110) de produit à diffuser, dont le col est introduit dans une tête (120) de diffusion communiquant avec l'intérieur dudit flacon. L'invention concerne plus particulièrement la diffusion de fragrances, de sorte que le produit contenu dans le flacon (110) est plus généralement un parfum ou une huile essentielle. Cependant, l'invention est également adaptée à la diffusion d'autres produits, notamment des produits pour la désinfection, pour la neutralisation des odeurs, l'humidification, la diffusion de produits répulsifs ou d'insecticides et d'une manière générale, la diffusion d'un produit sous la forme d'un aérosol. La tête (120) de diffusion est connectée à une arrivée (121) d'air comprimé, par exemple sous la forme d'un flexible. Cette tête de diffusion comporte une buse de nébulisation, laquelle est en communication avec l'intérieur du conduit d'un boîtier (130) anti-condensation par des moyens (122) appropriés, généralement un tube, rigide ou souple. Ledit boîtier (130) anti-condensation comprend un conduit qui, selon cet exemple de réalisation, est de section rectangulaire et constitué par un assemblage de tôles (131, 132) pliées soudées. L'extrémité (134) du conduit, proximale de l'arrivée (122) de produit nébulisé, comprend des moyens (140) pour la génération d'un flux d'air, par exemple, un ventilateur (140) électrique dit silencieux. Un tel ventilateur est couramment utilisé pour des applications telles que la ventilation d'une armoire électrique. À titre d'exemple non limitatif, ledit ventilateur a un diamètre de 80 mm, et délivre un débit de l'ordre de 40 m³/h pour une puissance électrique de l'ordre de 3 W. Ledit ventilateur est alimenté par une ligne très basse tension (non représentée) en 12 V ou en 24 V. Selon un mode de réalisation particulier, une résistance électrique (non représentée) alimentée par le même courant que le ventilateur, est placée dans le flux d'air immédiatement en aval dudit ventilateur (140) afin de réchauffer ledit flux d'air à une température de l'ordre de 30 °C. Cette température permet une meilleure diffusion des parfums sans les dénaturer.

Selon cet exemple de réalisation, le conduit est de section rectangulaire et cintré à 90 ° selon un faible rayon de courbure, de sorte à réduire son encombrement à longueur égale. Avantageusement, selon cet exemple de réalisation, la position du cintrage est choisie de sorte que le conduit entoure le flacon (110) et permette de diriger le jet vers le bas lorsque ledit flacon est en position verticale. Ce mode de réalisation est ainsi particulièrement compact et adapté à une utilisation où le dispositif objet de l'invention est dissimulé dans un faux plafond.

La section du conduit est déterminée par le jet de nébulisation débouchant dans ledit conduit. En effet, si la hauteur du conduit face à cette arrivée est trop faible, l'aérosol créé lors de la nébulisation tend à se condenser sur la paroi opposée à ladite arrivée de produit. À titre d'exemple non limitatif, la hauteur dudit conduit pour la diffusion d'un parfum est de l'ordre de 10 cm et plus généralement comprise entre 5 cm et 15 cm. La longueur du conduit est choisie pour obtenir, en sortie de celui-ci, un brouillard sec. Ladite longueur est déterminée, par exemple, au moyen d'essais en fonction du produit nébulisé. À titre d'exemple non limitatif, la longueur totale du conduit est comprise entre 3 fois et 10 fois le diamètre du cercle inscrit dans la section du conduit.

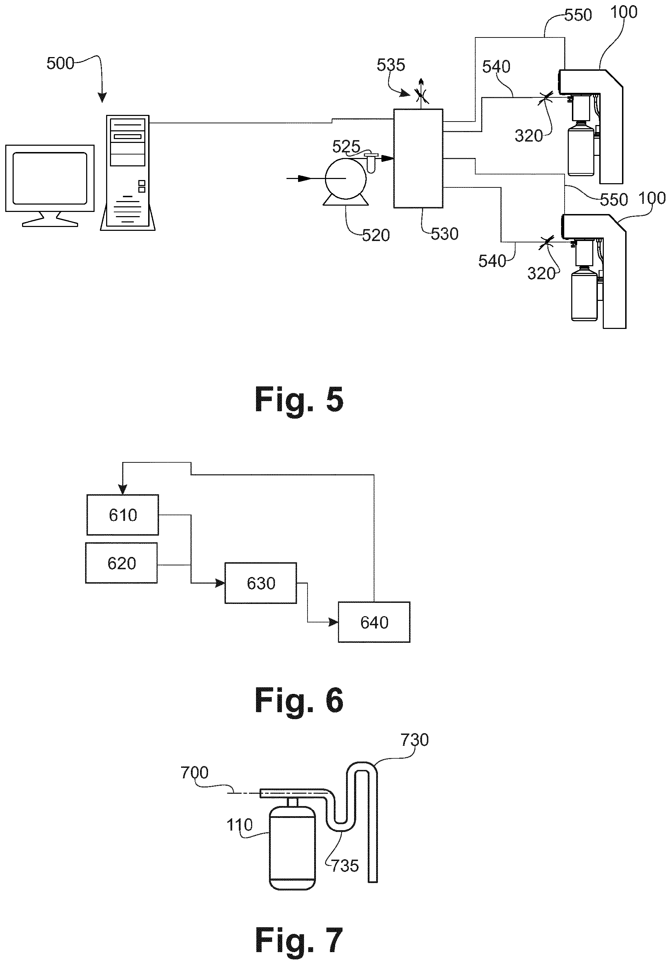

Figure 7, selon un exemple de réalisation ne faisant pas partie de l'invention, le conduit (730) du dispositif comprend un double cintre, de sorte que ledit conduit (730) comprend des surfaces s'étendant sensiblement perpendiculairement à la direction (700) originelle du flux d'air, et qui constituent ainsi des obstacles à la progression dudit flux. Les plus grosses gouttelettes sont projetées contre les parois du conduit au niveau des cintrages, où elles s'écoulent vers une zone (735) de rétention. Selon cet exemple de réalisation les cintrages sont réalisés autour d'axes sensiblement perpendiculaires à la direction (700) initiale du flux d'air et selon des rayons de courbure de l'ordre du diamètre du conduit. Un résultat équivalent est obtenu par un ou plusieurs cintrages selon un axe parallèle à cette direction (700) initiale du flux d'air et selon des rayons de courbures plus serrés. De même, si selon cet exemple de réalisation ne faisant pas partie de l'invention, le conduit est orienté de sorte que le flux d'air est orienté vers le bas à l'extrémité de diffusion, selon d'autres modes de réalisation, le conduit est orienté de sorte que cette diffusion ait lieu vers le haut, ou à l'horizontale ou selon toute orientation adaptée à la configuration de l'installation comprenant un tel dispositif. Selon d'autres exemples de réalisation, les coudes ainsi réalisés décrivent un angle inférieur ou supérieur à 90 °.

En revenant à la figure 1, selon cet exemple de réalisation, lorsque le produit nébulisé est introduit dans le conduit par l'arrivée (122) débouchant dans le boîtier anti-condensation, et que le produit ainsi nébulisé est transporté par le flux d'air produit par le ventilateur (140), les plus grosses gouttes, par exemple celles dont le diamètre est supérieur à 10 µm (10.10⁻⁶ m) seraient projetées contre la partie (133) cintrée du conduit. Toutefois, selon le mode d'installation de cet ensemble de diffusion, les gouttes ainsi projetées sur la partie (133) cintrée du conduit seraient susceptibles de couler le long des parois dudit conduit et de tomber par l'extrémité de diffusion. Afin d'éviter une telle situation, l'ensemble de diffusion objet de l'invention comporte, dans le conduit, une chicane (135) qui coopère avec les parois du conduit afin de créer une zone rétention des gouttes de produit nébulisé capturées par ladite chicane (135). La présence d'une telle chicane (135) n'est pas limitée à ce type de conduit. La hauteur de la chicane est adaptée en fonction de sa position dans le conduit de sorte à intercepter les plus grosses gouttelettes, et à laisser passer les gouttelettes plus fines, notamment dont le diamètre est inférieur à 10 µm. Le terme « chicane » s'entend d'un obstacle localisé dans le conduit, lequel obstacle est apte à dévier la progression du flux gazeux dans ledit conduit. Selon des exemples de réalisation non exhaustifs, une telle chicane est obtenue en rapportant une plaque fixée à l'intérieur du conduit par tout moyen tel que soudure, brasure, rivetage, ou est réalisé sous la forme d'une nervure venant de matière avec le conduit et réalisée par pliage ou par moulage.

Figure 2, selon cet exemple de réalisation de l'ensemble de diffusion objet de l'invention, la zone de rétention délimitée par la chicane dans le conduit, est en communication avec un récipient (210), par exemple au moyen d'un flexible (235). Selon cet exemple de réalisation, ledit récipient (210) est lié au boîtier anti-condensation au moyen d'un support (215), fixé à une des parois du conduit. Une autre paroi du conduit comporte avantageusement des pattes (250) de fixation pour suspendre ledit boîtier anti-condensation. Ainsi, selon cet exemple de réalisation, l'ensemble de diffusion (100) objet de l'invention forme un tout compact dont l'ensemble des moyens est lié au conduit de diffusion. Pour faire fonctionner cet ensemble il suffit d'un raccordement (121) à une source d'air comprimé, et d'un raccordement électrique (241) pour le ventilateur.

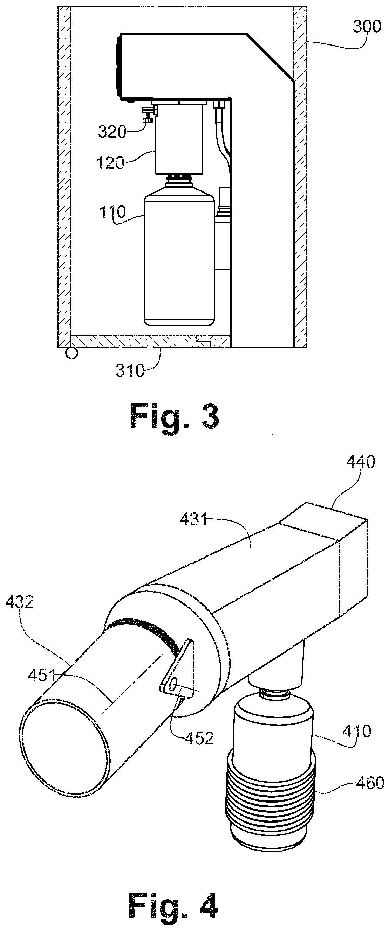

Figure 3, l'ensemble de diffusion objet de l'invention est avantageusement placé dans un habillage (300) comprenant une trappe (310) pour pouvoir accéder au flacon (110) afin de recharger ou de changer celui-ci lorsque tout le produit a été consommé. À cet effet, le flacon comprend avantageusement une jauge de niveau (non représentée), visuelle ou électronique permettant à un opérateur de maintenance de détecter le besoin d'un remplacement ou d'un remplissage. Selon cet exemple de réalisation ledit habillage (300) est conçu pour une installation de l'ensemble de diffusion au plafond. Dans cette situation, ladite trappe (310) permet également d'accéder au dispositif pour en assurer la maintenance. L'arrivée d'air comprimé dans la tête de diffusion (120) comprend avantageusement un moyen (320) pour régler le débit d'air injecté. Le réglage de ce débit détermine la quantité de produit aspiré par effet venturi dans le flacon (110) et ainsi, la quantité de produit nébulisé dans le boîtier anti-condensation. Selon cet exemple de réalisation le moyen (320) de réglage du débit est manuel. Alternativement il s'agit d'un moyen électronique piloté à distance.

Figure 4, selon un autre exemple de réalisation ne faisant pas partie de l'invention, le conduit de m'ensemble de diffusion comprend deux parties (431, 432) orientables l'une par rapport à l'autre, ici selon deux liaisons pivot d'axes (451, 452) perpendiculaires. Ainsi, la partie (432) comprenant l'extrémité de diffusion du conduit est orientable afin d'optimiser la direction du flux de diffusion par rapport à l'environnement dans lequel se trouve cet ensemble de diffusion. Selon cet exemple de réalisation ne faisant pas partie de l'invention, le ventilateur (440) est connecté à la partie (431) fixe du conduit laquelle est également connectée au flacon (410) contenant le produit à diffuser. Selon un mode de réalisation particulier, non représenté, l'orientation de la partie (432) mobile par rapport à la partie (431) fixe du conduit est réalisée par des moyens de motorisation pilotés à distance.

Selon un mode de mise en œuvre, ne faisant pas partie de l'invention, l'ensemble de diffusion objet de l'invention comporte un moyen (460) de chauffage du flacon (410) de produit à diffuser. Selon cet exemple de réalisation, non exclusif, ledit moyen de chauffage consiste en une résistance électrique (460) apte à chauffer le flacon par l'extérieur. D'autres modes de chauffage sont possibles par l'extérieur du flacon ou directement dans le produit. Ainsi, lorsque l'ensemble de diffusion objet de l'invention est utilisé dans un environnement froid, ces moyens (460) de chauffage permettent d'amener le produit diffusé dans des conditions de viscosité favorisant sa nébulisation. Selon la nature du produit diffusé, les moyens de chauffage permettent aussi de favoriser la vaporisation du produit lors de son passage dans la tête de diffusion.

Figure 5, selon un exemple de réalisation ne faisant pas partie de l'invention du dispositif de diffusion, adapté notamment à la diffusion dans un grand volume, ledit dispositif comprend une pluralité d'ensembles (100) de diffusion alimentée en air comprimé par un bloc (520) pompe unique. Selon des variantes de réalisation, ledit bloc (520) pompe est constitué d'un compresseur à piston, d'un compresseur à vis ou de tout autre dispositif apte à produire de l'air comprimé, associé ou non à une capacité tampon et avantageusement constitué d'un compresseur à membrane ou à piston oscillant fonctionnant sans huile. En sortie du bloc (520) pompe, l'air comprimé est dirigé vers un bloc (530) de répartition, en passant par un filtre ou un sécheur déshuileur (525). Ledit bloc (530) de répartition de l'air comprimé comprend un régulateur (535) de pression, à réglage manuel ou à pilotage électronique, permettant, par exemple, par la création d'une fuite, d'adapter la pression issue du bloc pompe, généralement 8 bars, au nombre d'ensembles (100) de diffusion alimentés. Ainsi, quel que soit le nombre d'ensembles de diffusion (100) connectés, la pompe (520) fonctionne toujours dans des conditions favorables, ce qui évite son usure prématurée et réduit le niveau sonore.

Selon cet exemple de réalisation ne faisant pas partie de l'invention, des moyens (500) de supervision et de pilotage permettent de piloter le bloc (530) de répartition ou le bloc pompe et ainsi de programmer, pour chaque ensemble (100) de diffusion, la durée de nébulisation, la quantité de produit nébulisée et la fréquence temporelle de ces diffusions en fonction des caractéristiques de l'espace dans lequel la diffusion a lieu. L'air comprimé est injecté dans les têtes de diffusion sous une pression de l'ordre de 0,6 bar, plus généralement entre 0,3 bar et 1,5 bar selon le produit diffusé et le résultat visé. Selon un exemple de réalisation, le bloc (530) de répartition pilote l'envoi d'air comprimé dans les canalisations (540) reliant ledit bloc (530) aux ensembles (100) de diffusion. Selon un autre exemple de réalisation, le bloc de répartition commande également la mise en route et l'arrêt des ventilateurs des ensembles de diffusion. Dans ce dernier exemple de réalisation ne faisant pas partie de l'invention, le bloc de répartition est connecté à chaque ensemble (100) de diffusion par une liaison (540) pneumatique et par une liaison (550) électrique en très basse tension.

Les ensembles (100) de diffusion sont répartis dans l'espace dans lequel le produit doit être diffusé. Cette répartition est obtenue par des essais ou par des simulations. Ces essais ou ces simulations préalables à la mise en œuvre du dispositif objet de l'invention, permettent également de définir la fréquence temporelle des nébulisations et le volume de produit ainsi nébulisé. Le volume de produit nébulisé dépend du calibrage de la pression et du débit d'air injectés dans les têtes de diffusion des ensembles (100) de diffusion. Ces réglages sont réalisés, d'une part, au moyen du régulateur (535) de pression, et d'autre part, au moyen des dispositifs (320) individuels de réglage , représenté ici schématiquement, de chaque ensemble (100) de diffusion.

Selon un exemple de réalisation, lorsque le dispositif de diffusion ne comprend qu'un seul ensemble de diffusion, l'utilisation d'un bloc de répartition n'est pas nécessaire. Le régulateur de pression est installé en sortie du bloc pompe et les moyens (500) de supervision pilotent directement le bloc pompe et le ventilateur dudit ensemble (100) de diffusion.

Figure 6, selon un exemple de réalisation, un procédé de mise en oeuvre ne faisant pas partie de l'invention, comprend à l'échelle d'un ensemble de diffusion, une première étape (610) d'injection pilotée et consistant à injecter de l'air comprimé sous une pression adaptée dans la tête de diffusion dudit ensemble de diffusion. Le pilotage de cette étape est réalisé par les moyens de supervision agissant sur le bloc de répartition ou directement sur le bloc pompe. Simultanément à cette étape d'injection, une étape (620) de ventilation consiste à piloter la mise en route du ventilateur de l'ensemble de diffusion. Cette étape (620) de ventilation est, selon le produit diffusé et les conditions de mise en œuvre, parfaitement simultanée à la nébulisation ou prédéclenchée avant la nébulisation ou encore déclenchée selon une temporisation donnée après ladite nébulisation. Selon une étape (630), dite de post-ventilation, du procédé objet de l'invention, la ventilation est poursuivie après que l'étape de nébulisation soit achevée. Selon la nature des moyens de ventilation, l'étape (630) de post-ventilation est réalisée avec un débit d'air identique à celui de l'étape (620) de ventilation ou avec un débit différent. De plus, lorsque l'ensemble de diffusion est pourvu d'un moyen de chauffage du flux d'air, la température de ce flux d'air est également contrôlée au cours de ces deux étapes (620, 630). En effet, l'étape (620) de ventilation a pour objectif de créer un flux de brouillard sec à partir du nuage de produit nébulisé dans le boîtier anti-condensation, alors que l'étape (630) de post-ventilation a pour objectif de faire évaporer les gouttes de produit qui se sont déposées sur les parois du conduit au cours des étapes précédentes, ainsi les conditions de ventilation et de chauffage correspondant à ces deux étapes (620, 630) sont spécifiquement adaptées à l'objectif visé. Après une étape (640) de temporisation, spécifique à chaque ensemble de diffusion piloté, le processus est repris.

La description ci-avant et les exemples de réalisation montrent que l'invention atteint les objectifs visés, en particulier le dispositif objet de l'invention permet de diffuser un produit de manière optimale et contrôlée dans un espace, quel que soit le volume de cet espace, par une répartition des ensembles de diffusion dans cet espace. Le boîtier anti-condensation objet de l'invention permet :
- de diluer le brouillard de produit dans un plus gros volume d'air sec ;
- de faciliter l'évaporation du produit avant sa sortie du conduit et sa diffusion dans l'espace objet de la diffusion de produit ;
- d'améliorer la finesse du brouillard diffusé dans un volume global compact ;
- de retenir les gouttes de liquide se formant à la sortie de la tête de diffusion ;
- de récupérer les excédents de produit liquide s'étant formés à la sortie de la tête de diffusion et d'éviter les contaminations ;
- de guider le flux d'air - produit vers des zones spécifiques du volume de diffusion ;
- d'augmenter la vitesse du flux d'air - produit et ainsi améliorer la répartition du produit dans le volume objet de la diffusion.

## Revendications

1. Boîtier (130), dit anti-condensation, comprenant des moyens pour être connecté à une tête (120) de diffusion apte à produire une nébulisation d'un produit à diffuser contenu dans un flacon (110, 410), **caractérisé en ce qu'**il comporte :
a. un conduit (131, 132, 431, 432, 730) comportant une partie cintrée (133, 432) et une arrivée (122) du produit nébulisé dans le conduit, par une buse de nébulisation entre une extrémité de diffusion et une extrémité d'insufflation;
b. à l'extrémité de diffusion dudit conduit, un orifice de sortie ;
c. à l'extrémité (134) d'insufflation dudit conduit un ventilateur (140, 440) pour générer un flux d'air entre ladite extrémité et l'extrémité de diffusion du conduit ;
d. une chicane (135) à l'intérieur dudit conduit, située entre l'arrivée (122) du produit nébulisé et l'extrémité de diffusion, laquelle chicane s'étend selon une direction perpendiculaire à la direction du flux d'air de sorte à créer un obstacle dans la progression du flux dans le conduit entre la buse de nébulisation et la partie cintrée (133, 432) ;
e. la chicane (135) coopérant avec la paroi (132) dudit conduit pour créer dans ledit conduit une zone de rétention du produit nébulisé arrêté par ladite chicane.

2. Boîtier selon la revendication 1, dans lequel le diamètre du cercle inscrit dans la section du conduit (131, 132, 431, 432) est compris entre 60 mm et 120 mm.

3. Boîtier selon la revendication 1, dans lequel la longueur du conduit entre l'extrémité (134) d'insufflation et l'extrémité de diffusion est comprise entre 3 fois et 10 fois le diamètre du cercle inscrit dans la section du conduit..

4. Boîtier selon la revendication 1, comprenant :
g. un récipient (210) en communication avec la zone de rétention

5. Boîtier selon la revendication 1, comprenant :
h. des moyens pour chauffer le flux d'air généré à l'extrémité (134) d'insufflation.

6. Dispositif pour la diffusion d'un produit sous la forme d'un brouillard sec, **caractérisé en ce qu'**il comporte un ensemble de diffusion comprenant :
i. un flacon (110, 410) contenant le produit à nébuliser ;
ii. une tête (120) de diffusion, laquelle tête est connectée au flacon et comprend une arrivée (121) d'air comprimée ;
iii. un boîtier anti-condensation selon la revendication 1, dont l'arrivée (122) de produit nébulisé est connectée à la tête de diffusion.

7. Dispositif selon la revendication 6, dans lequel la tête (120) de diffusion comprend des moyens (320) réglables pour limiter le débit d'air comprimé injecté dans ladite tête de diffusion.

8. Dispositif selon la revendication 6, dans lequel le conduit de l'ensemble de diffusion comprend des moyens (250) de fixation pour la suspension dudit conduit et du flacon (110).

9. Dispositif selon la revendication 6, comprenant :
vi. un habillage (300) comprenant une trappe (310) comportant une serrure, et un orifice communiquant avec l'extrémité de diffusion du conduit du boîtier anti-condensation.

## Patentansprüche

1. Gehäuse (130), Anti-Kondensationsgehäuse genannt, welches Mittel umfasst, um an einen Diffusionskopf (120) angeschlossen zu werden, der imstande ist, eine Zerstäubung eines zu diffundierenden Produkts zu produzieren, das in einem Flakon (110, 410) enthalten ist, **dadurch gekennzeichnet, dass** es beinhaltet:
a. eine Leitung (131, 132, 431, 432, 730), die einen gebogenen Teil (133, 432) und eine Zuleitung (122) des zerstäubten Produkts in die Leitung durch eine Zerstäubungsdüse zwischen einem Diffusionsende und einem Einblasende beinhaltet;
b. am Diffusionsende der Leitung eine Ausgangsöffnung;
c. am Einblasende (134) der Leitung einen Lüfter (140, 440) zum Erzeugen einer Luftströmung zwischen dem Ende und dem Diffusionsende der Leitung;
d. eine Schikane (135) im Inneren der Leitung, die sich zwischen der Zuleitung (122) des zerstäubten Produkts und dem Diffusionsende befindet, wobei sich die Schikane in einer Richtung senkrecht zur Richtung der Luftströmung erstreckt, um ein Hindernis in der Ausbreitung der Strömung in der Leitung zwischen der Zerstäubungsdüse und dem gebogenen Teil (133, 432) zu schaffen;
e. wobei die Schikane (135) mit der Wand (132) der Leitung zusammenwirkt, um in der Leitung eine Rückhaltezone des von der Schikane angehaltenen zerstäubten Produkts zu schaffen.

2. Gehäuse nach Anspruch 1, wobei der Durchmesser des Kreises, der in den Querschnitt der Leitung (131, 132, 431, 432) eingeschrieben ist, zwischen 60 mm und 120 mm enthalten ist.

3. Gehäuse nach Anspruch 1, wobei die Länge der Leitung zwischen dem Einblasende (134) und dem Diffusionsende zwischen 3 Mal und 10 Mal dem Durchmesser des Kreises, der in den Querschnitt der Leitung eingeschrieben ist, enthalten ist.

4. Gehäuse nach Anspruch 1, umfassend:
g. einen Behälter (120) in Verbindung mit der Rückhaltezone.

5. Gehäuse nach Anspruch 1, umfassend:
h. Mittel zum Erhitzen der am Einblasende (134) erzeugten Luftströmung.

6. Vorrichtung zur Diffusion eines Produkts in Form eines Trockennebels, **dadurch gekennzeichnet, dass** sie eine Diffusionsbaugruppe beinhaltet, die Folgendes umfasst:
i. einen Flakon (110, 410), der das zu zerstäubende Produkt enthält;
ii. einen Diffusionskopf (120), welcher Kopf an den Flakon angeschlossen ist und eine Zuleitung (121) für Druckluft umfasst;
iii. ein Anti-Kondensationsgehäuse nach Anspruch 1, dessen Zuleitung (122) für das zerstäubte Produkt an den Diffusionskopf angeschlossen ist.

7. Vorrichtung nach Anspruch 6, wobei der Diffusionskopf (120) einstellbare Mittel (320) umfasst, um den Druckluftdurchsatz, der in den Diffusionskopf injiziert wird, zu begrenzen.

8. Vorrichtung nach Anspruch 6, wobei die Leitung der Diffusionsbaugruppe Befestigungsmittel (250) für die Aufhängung der Leitung und des Flakons (110) umfasst.

9. Vorrichtung nach Anspruch 6, umfassend:
vi. eine Verkleidung (300), umfassend eine Klappe (310), die ein Schloss beinhaltet, und eine Öffnung, die mit dem Diffusionsende der Leitung des Anti-Kondensationsgehäuses verbunden ist.

## Claims

1. Anti-condensation housing (130), comprising means for being connected to a diffusion head (120) able to produce nebulisation of a product to be diffused contained in a bottle (110, 410), **characterised in that** it comprises:
a. a conduit (131, 132, 431, 432, 730) comprising a curved part (133, 432) and an inlet (122) for the nebulised product into the conduit, through a nebulisation nozzle between a diffusion end and a blowing end;
b. at the diffusion end of said conduit, an outlet orifice;
c. at the blowing end (134) of said conduit a fan (140, 440) for generating an air flow between said end and the diffusion end of the conduit;
d. a baffle (135) inside said conduit, situated between the inlet (122) for the nebulised conduit and the diffusion end, said baffle extending in a direction perpendicular to the direction of the air flow so as to create an obstacle in the progress of the flow in the conduit between the nebulisation nozzle and the curved part (133, 432);
e. the baffle (135) cooperating with the wall (132) of said conduit in order to create in said conduit a retention zone for the nebulised product stopped by said baffle.

2. Housing according to claim 1, wherein the diameter of the circle inscribed in the cross section of the conduit (131, 132, 431, 432) is between 60 mm and 120 mm.

3. Housing according to claim 1, wherein the length of the conduit between the blowing end (134) and the diffusion end is between three times and ten times the diameter of the circle inscribed in the cross section of the conduit.

4. Housing according to claim 1, comprising:
g. a receptacle (210) in communication with the retention zone.

5. Housing according to claim 1, comprising:
h. means for heating the air flow generated at the blowing end (134).

6. Device for diffusing a product in the form of a dry mist, **characterised in that** it comprises a diffusion assembly comprising:
i. a bottle (110, 410) containing the product to be nebulised;
ii. a diffusion head (120), said head being connected to the bottle and comprising a compressed-air inlet (121);
iii. an anti-condensation housing according to claim 1, wherein the inlet (122) for the nebulised product is connected to the diffusion head.

7. Device according to claim 6, wherein the diffusion head (120) comprises adjustable means (320) for limiting the compressed-air flow injected into said diffusion head.

8. Device according to claim 6, wherein the conduit of the diffusion assembly comprises fixing means (250) for suspending said conduit and the bottle (110).

9. Device according to claim 6, comprising:
vi. a casing (300) comprising a hatch (310) comprising a lock, and an orifice communicating with the diffusion end of the conduit of the anti-condensation housing.
